# EUROPEAN PATENT SPECIFICATION

(11) **EP 2 887 868 B1**
(45) Date of publication and mention of the grant of the patent: **07.02.2018**
(21) Application number: 12761919.5
(22) Date of filing: 21.08.2012
(51) Int. Cl.: A61B 5/153, A61B 5/154, A61B 5/155, A61B 5/15, A61B 5/00

(54) **BLOOD COLLECTION NEEDLE ASSEMBLY HAVING A LIGHT SOURCE**
BLUTENTNAHMENADELANORDNUNG MIT EINER LICHTQUELLE
ENSEMBLE AIGUILLE DE PRÉLÈVEMENT SANGUIN COMPORTANT UNE SOURCE LUMINEUSE

(43) Date of publication of application: 01.07.2015
(73) Proprietor: Optomeditech Oy, 00100 Helsinki (FI)
(72) Inventor: GASPARYAN, Levon, FI-00270 Helsinki (FI); ROMBERG, Hans, 76297 Stutensee (DE)
(74) Representative: Hutter, Anton
(86) International application number: PCT/EP2012/066243
(87) International publication number: WO 2014/029421

(56) References cited:
- US-A- 4 311 138
- US-A- 5 030 207
- US-A1- 2002 115 922
- US-A1- 2007 073 160
- US-A1- 2009 149 771
- US-A1- 2011 009 738

## Description

### FIELD OF THE INVENTION

The present invention relates to blood collection tools and, in particular, to a blood collection needle, known also as phlebotomy needle, assembly, comprising means for instantaneous determination of blood vessel penetration moment and ensuring a correct position of the blood collection needle inside blood vessel.

### BACKGROUND

Venipuncture is one of the most routinely performed invasive medical procedures that are commonly used for the collection of blood samples. However, regardless of the wide utilization of said procedure in medical practice, medical personnel often face certain problems while performing venipuncture. At first, whenever a patient has small, deep, faulty or damaged blood vessels it is difficult to visually localize, palpate, and reach thereof. A clinician ends up with multiple trials of venipuncture that cause unnecessary pain and stress to a patient and that are unacceptable in emergency situations when time is mostly critical. Another major problem is difficulty in determination of the exact position of a needle tip inside the blood vessel, wherein a clinician in certain cases is unable to determine whether the blood vessel is punctured correctly and whether the needle tip is properly localized inside the vein. The most widely utilized method for determination of the exact moment of intravenous penetration is to follow blood flow into a blood collection tube, connectable to a blood collection needle. The method, however, works poorly on patients, whose blood vessels to be punctured, herein veins, are difficult to reach.

At present, several techniques are used in order to locate blood vessels or to facilitate the puncture of blood vessels.

US 2009/0043225 discloses a self-venting blood collection needle assembly for the extraction of fluid sample into an evacuated container for laboratory testing. This assembly is provided with a clear or translucent flashback chamber to visually detect successful vein entry for a user.

US 2009/0088698 discloses a blood collection needle assembly for flashback detection during a blood collection procedure. Said needle assembly includes a housing with a base portion defining a cavity and a transparent extension extending distally from the base portion and defining a bore therethrough.

US 2008/0167577 discloses a phlebotomy needle with means for flashback visualization. This device comprises a needle and housing with a passive vent and a viewing region for visual detection of blood flow into the housing.

US 7,918,805 discloses a phlebotomy device that includes a temperature sensor disposed at least partially within the lumen of a needle. The sensor is configured to change its shape from a first state to a second state upon exposure to blood at physiological temperature.

US 5,954,701 discloses a blood vessel entry indicator comprising a needle and a pressure sensor that is arranged so as to be able to activate sound signal emitting means. A sound signal is emitted when the needle enters a blood vessel.

Various means for blood vessel entry detection, disclosed e.g. in US 2009/149771, US 5,314,410, US 2007/073160, US 4,971,068, US 2011/009738, US 4,311,138 and US 5,030,207, often require additional equipment and do not provide an instantaneous evidence of successful entry into a blood vessel.

It is therefore still desirable to provide a simple, fast, safe, reliable and portable needle device for venipuncture and blood collection, said device comprising means for instantaneous determination of blood vessel penetration moment. It is further desirable that said device would not require any additional visualization equipment.

### SUMMARY OF THE INVENTION

This object is achieved, in accordance with the present invention, by a blood collection needle assembly comprising a tubular needle having a distal end, a proximal end and a tube wall that defines a lumen of the needle. A blood entry aperture is formed at the distal end of the needle, and the blood exit aperture is formed at the proximal end of the needle. The assembly further comprises a tube holder releasably or permanently attached to the needle and a light source configured to emit light and releasably and/or permanently attached to or integrated in the tube holder. The tube wall of the needle has a light entry aperture which is arranged at a position between the blood entry aperture and the blood exit aperture. The light emitted by the light source is allowed to enter the lumen of the needle through the light entry aperture.

The provision of a light entry aperture in the tube wall of the needle enables light emitted by the light source to propagate through the lumen of the needle and to emerge from its distal end. If the distal end of the needle enters a blood vessel, the light shining through the tissue changes its intensity and/or colour, which can be detected by an observer or an external detector. Thus the blood collection needle assembly of the present invention ensures correctly performed venipuncture in a minimal time frame and helps to avoid multiple punctures caused by failure in blood vessel localization, as well as blood vessel through punctures. The blood collection needle assembly of the invention is of particular importance for use in emergency cases, ambulance as well as in children's hospitals.

Generally it is preferred if the light emitted by the light source is visible light. Then any change in light colour or light intensity can be directly observed without a need for additional detectors. In that case the tube wall of the needle should be, except for the light entry aperture, non-transparent for visible light so that only light emerging from the distal end of the tubular needle contributes to the visual impression of the observer.

The change of intensity is particularly strong if the light emitted by the light source has a strong yellow and/or green colour component. For example, at least 40 % of light contributing to the total light intensity emerging from the distal end of the needle may have a wavelength either between 510 nm and 600 nm. Light having these wavelengths is particularly strongly absorbed by blood, and thus the moment of blood vessel penetration can be observed easily by the sudden disappearance of light that was visible before from the skin of the patient.

The blood collection needle assembly may comprise an elastic blood stopper which is attached to the proximal end of the needle so that it covers a portion of the needle. Such an elastic blood stopper forms a kind of vent which allows blood to penetrate the blood stopper once it is punctured by a cap of a blood collection tube, but does not allow blood to penetrate the blood stopper if no blood collection tube is supplied. The proximal end of the needle may be configured to puncture the elastic blood stopper if the latter is pressed against the proximal end by a blood collection tube. To this end the proximal end of the needle may be sharpened. For example, the proximal end of the needle may be formed by an oblique cut plane that is inclined with respect to a longitudinal axis of the needle by an angle between 10° and 80°.

The distal end of the needle is usually also configured to puncture a blood vessel and may be sharpened to this end. Sharpening may be achieved or supported if the needle is formed by an oblique cut plane that is inclined with respect to a longitudinal axis of the needle by an angle between 10° and 80°.

In some embodiments at least a portion of the tube wall of the needle is made of a metal.

The blood collection needle assembly may comprise an adaptor lock which connects a needle with the tube holder. The adaptor lock may be manufactured from a material that is non-transparent for the light emitted by the light source. However, also semi-transparent or completely transparent or at least translucent materials may be used for the adaptor lock or at least for a portion thereof.

Generally the light entry aperture may be open so that light may enter the lumen of the needle, but also blood is capable of leaving the lumen via the light entry aperture. In that case an additional chamber should be provided outside the lumen of the needle for collecting blood that passed through the open light entry window.

Generally, however, it is preferred that the light entry aperture is closed by a non-diffusing light entry member that is transparent for at least some of the wavelengths of light emitted by the light source.

A particularly effective way to guide light from the light source to the distal end of the needle involves the use of a light guide, for example an optical fiber. Then at least a portion of the light entry member is formed by the light guide that extends through the light entry aperture.

In order to guide the light towards the distal end of the needle, the light guide should further extend along the lumen towards the distal end of the needle, and preferably extend at least between the light entry aperture and the distal end of the needle.

If the needle is completely straight, a light guide extending through the light entry aperture will have to be bent by an angle between 60° and 175° inside the lumen. Generally the lumen may have any arbitrary cross section, in particular a circular or a polygonal cross section.

The needle may be formed as a one-piece hollow metallic needle, but it may also comprise separate metallic or non-metallic portions that are joined together by at least one non-metallic portion. The latter may be formed by the adaptor lock which connects the needle to the tube holder.

In some embodiments the needle has a slanted middle section, which may be formed by the non-metallic portion that joins separate portions of the needle. If the light entry aperture is then positioned in the slanted middle section of the needle, this facilitates the lateral coupling of light into the lumen and avoids strong bends of a light guide, for example. In that case the light entry aperture is preferably positioned at least substantially opposite the blood entry aperture so that light entering the lumen through the light entry aperture is able to propagate along a straight line through the lumen towards the blood entry aperture of the needle. Then sufficiently high light intensities may be achieved even without an optical fiber utilizing reflection of light from the tube walls of the needle.

In some embodiments the tube wall of the needle includes a light exit window that is translucent or transparent for light emitted by the light source and is arranged at least substantially opposite to the light entry aperture. This light exit window then serves as an additional flash back indicator that indicates that blood has entered the lumen of the needle.

In order to illuminate the light exit window, a further light guide may be provided that also extends through the light entry aperture and has a distal end that points towards the light exit window.

In some embodiments the adapter lock may comprise an elongated distal section, which forms a flashback chamber to observe an optical fiber located inside said section. Inner volume, thus created by inner walls of a flashback chamber, forms a lumen, provided as a part of the lumen of the needle. Said distal section is preferably made from a transparent material. An external observer may therefore see a light guide as well as optical effects related to illumination of the light guide and detect blood after successful venipuncture.

In other embodiments the tube wall of the needle includes a venting mechanism. Such a venting mechanism may comprise a porous filter member which allows an air escape, but blocks an outflow of blood to ambient surroundings. If the filter member is itself translucent for visible light, it may form the light exit window that is used as a flash back indicator.

The light source as well as other optical and electronic components should be attached to or integrated in those parts of the assembly which shall not be disposed after use. For that reason the light source is attached to or integrated in the tube holder which is usually releasably attached to the needle so that it is suitable for multiple use. However, it may be necessary to replace also the tube holder often. In order to preserve the valuable optical and electronic components associated with the light source, it may be envisaged to attach the light source releasably to an outer surface of the tube holder. Then the light source can be used even if the tube holder has to be replaced.

In that case the blood collection needle assembly needs to comprise a coupling connector attached to the light guide and configured to be releasably connected to an external light guide which is optically connected to the light source. Such a coupling connector may be realized as a conventional fiber optic connector, if the tube holder has a tubular side wall and a front wall covering the tubular side wall at one end thereof, the light guide may have a portion that extends through the front wall of the tube holder and terminating at the coupling connector. The latter may then comprise a coupling mechanism in the form of a hollow element incorporated within the front wall of the tube holder and adapted to receive the external light guide.

According to another aspect of the present invention, a blood collection needle assembly comprises a tubular needle having a distal end, a proximal end and a tube wall that defines a lumen of the needle, a tube holder attached to the needle, and a light source configured to emit light and arranged inside the lumen of the needle. In this case light is not coupled into the lumen of the needle via an aperture in its tube wall, but the light source is directly arranged inside the lumen.

The subject of the invention is also a blood collection needle for a blood collection needle assembly wherein the needle comprises a distal end, a proximal end, a tube wall that defines a lumen of the needle, a blood entry aperture formed at the distal end of the needle, a blood exit aperture formed at the proximal end of the needle, a light entry aperture which is arranged at a position between the blood entry aperture and the blood exit aperture, and a light guide that extends through the light entry aperture and along at least a portion of the lumen towards the blood exit aperture.

Preferably the light guide extends along the entire distance between the light entry aperture and the blood exit aperture of the needle.

Particularly if the light guide is an optical fiber, it may have a portion that is bent by an angle between 60° and 175°.

The blood collection needle may comprise an adaptor lock which is configured to collect the needle to a tube holder.

The term "light" refers in this disclosure to electromagnetic radiation comprising wavelengths in a range extending between a few nanometers (far ultraviolet spectral range) to a few thousand nanometers (far infrared spectral range). The term "visible light" refers to light that can be seen by the human eye. Typically this includes wavelengths between about 400 nm and 750 nm.

The term "blood vessel" in this disclosure is vastly equivalent to the term "vein", since peripheral veins are the most common access point for intravascular methods. To those skilled in art, however, it must be clear, that the term "blood vessel" may also relate to arteries.

The term "distal" refers in this disclosure to an end pointing away from a user and towards a patient. In case of one-piece blood collection needle, said term may also refer to an insertion end (tip) of the needle.

The term "proximal" refers in this disclosure to an end pointing towards a user and away from a patient.

Different embodiments of the present invention will become apparent by consideration of the detailed description and accompanying drawings.

### BRIEF DESCRIPTION OF THE DRAWINGS

Fig. 1A illustrates a conventional blood collection needle with a male threaded connector and an elastic blood stopper.
Fig. 1B illustrates a conventional blood collection needle assembly comprising a hollow metallic needle with a male threaded connector and an elastic blood stopper, together with a tube holder.
Fig. 1C illustrates conventional blood collection needle assembly with a blood collection tube attached thereto.
Figs. 2A-C illustrate a blood collection process by means of conventional blood collection needle assembly.
Fig. 3A schematically illustrates a blood collection needle assembly of the invention.
Fig. 3B schematically illustrates a distal portion of a blood collection needle of a blood collection needle assembly of the invention.
Figs. 3C and 3D illustrate a process of blood vessel penetration by means of the blood collection needle assembly of the invention.
Fig. 4A illustrated a single unit blood collection needle of the invention.
Fig. 4B illustrates the blood collection needle of the invention comprised of two separate units, connected by the adapter lock.
Fig. 5A illustrates the blood collection needle assembly (tube holder is not shown) of the invention, wherein said assembly comprises a hollow metallic blood collection needle with a slanted middle section, in accordance with some embodiment.
Fig. 5B illustrate the blood collection needle assembly (tube holder is not shown) of the invention comprised of two separate units, joined by the adapter lock, wherein said units are shifted related to each other, in accordance with some embodiment.
Figs. 6A and 6B illustrate the blood collection needle assembly (tube holder is not shown) of the invention, wherein said assembly comprises one and two light guides, respectively; and wherein an additional aperture is arranged in the tube wall of the blood collection needle, in accordance with the embodiments of the invention.
Fig. 6C illustrate the blood collection needle assembly (tube holder is not shown) of the invention, wherein said assembly is provided with a transparent elongated distal section of the adapter lock.
Figs. 7A and 7B is a schematic illustration of an event of light absorption by blood propagating along the lumen of the blood collection needle assembly of Fig.6B.
Figs. 7C and 7D is a schematic illustration of an event of light absorption by blood propagating along the lumen the blood collection needle assembly of Fig.6C.
Figs. 8A and 8B illustrate venting mechanism provided in the form of the second additional aperture in the wall of the needle assembly, said aperture sealed with a porous air-permeable material, in accordance with some embodiment.
Figs.8C and 8D illustrate venting mechanism provided in the form of the additional aperture in the wall of the needle assembly, said aperture sealed with a semitransparent porous air-permeable material, in accordance with some embodiment.
Fig. 9A illustrates a blood collection needle assembly with the light guide and the light source incorporated within the part of the tube holder, in accordance with some embodiment.
Fig. 9B illustrates a blood collection assembly with the light guide incorporated within the part of the tube holder and the light source arranged externally to the tube holder, in accordance with some embodiment (light source is not shown).
Fig. 9C illustrates a blood collection assembly with the light guide and the hollow connector incorporated within the part of the tube holder, in accordance to some embodiment.
Fig. 9D illustrates a blood collection needle assembly with the light guide incorporated within the part of the tube holder and the light source arranged within a separate unit, said unit preferably fixed to the tube holder.
Fig. 10A illustrates a blood collection needle assembly without a light guide, said assembly is provided with an additional optical element, arranged inside the lumen of the blood collection needle, and with the light source, arranged externally to the blood collection needle, in accordance with some embodiment.
Fig. 10B illustrates a blood collection needle assembly without a light guide; said assembly comprises a blood collection needle with a slanted middle section, and is provided with the light source, arranged externally to the blood collection needle in accordance with some embodiment.
Fig. 10C illustrates a blood collection needle assembly without a light guide; said assembly comprises a blood collection needle provided with the light source, incorporated inside the wall of the blood collection needle in accordance with some embodiment.
Fig. 10D illustrates a blood collection needle assembly with a light guide; said assembly comprises a two-unit blood collection needle provided with the light source, arranged inside the lumen of the blood collection needle in accordance with some embodiment.
Figs. 11A illustrates an exemplary blood collection needle assembly, where the light guide at least partially forms an integral part with the distal portion of the blood collection needle.
Fig. 11B illustrates an exemplary blood collection needle assembly comprised of two units, wherein the distal tubular unit of the blood collection needle is manufactured from a material alternative to metal.

### DETAILED DESCRIPTION OF THE EMBODIMENTS

Detailed embodiments of the present invention are disclosed herein with the reference to accompanying drawings. The same reference numerals are used throughout the drawings to refer the same members. The following reference numerals are used for the members:

### Prior art:

101 - blood collection needle assembly;
102 - hollow metallic blood collection needle;
103 - lumen of a blood collection needle;
104 - distal end (tip) of a blood collection needle;
105 - proximal end of a blood collection needle, facing a tube holder;
106 - male threaded connector of a blood collection needle;
107 - elastic blood stopper of a blood collection needle;
108 - tube holder;
121 - blood collection vacuum tube;
122 - blood collection tube cap;
131 - blood.

### Present disclosure:

201 - blood collection needle assembly;
202 - hollow blood collection needle;
202a, 202b - separate tubular units of the blood collection needle;
203 - lumen of the blood collection needle;
203a - lumen created by inner walls of the adapter lock in between two hollow units of the blood collection needle;
204 - distal end of the blood collection needle;
204a - blood entry aperture;
205 - proximal end of the blood collection needle, facing a tube holder;
206 - adapter lock of the blood collection needle;
206a - transparent section for the adapter lock;
207 - elastic blood stopper of the blood collection needle;
208 - tube holder;
210 - light guide;
211 - aperture in the wall of the blood collection needle (light entry aperture);
213 - additional aperture ("window") in the wall of the blood collection needle assembly;
214 -additional aperture in the wall of the blood collection needle assembly, said aperture sealed with a porous, air-permeable material ("venting mechanism");
215 - additional optical element;
220 - light source;
221 - light converging device;
231 - blood;
234 - coupling connector for connecting an external light guide;
235 - coupling mechanism incorporated within a front wall of the tube holder;
240 - external unit for the light source;
250 - light;
250a - light visible through the additional aperture 213 and/or the transparent section of the adapter lock 206a;
301 - blood collection needle with the lock adapter.

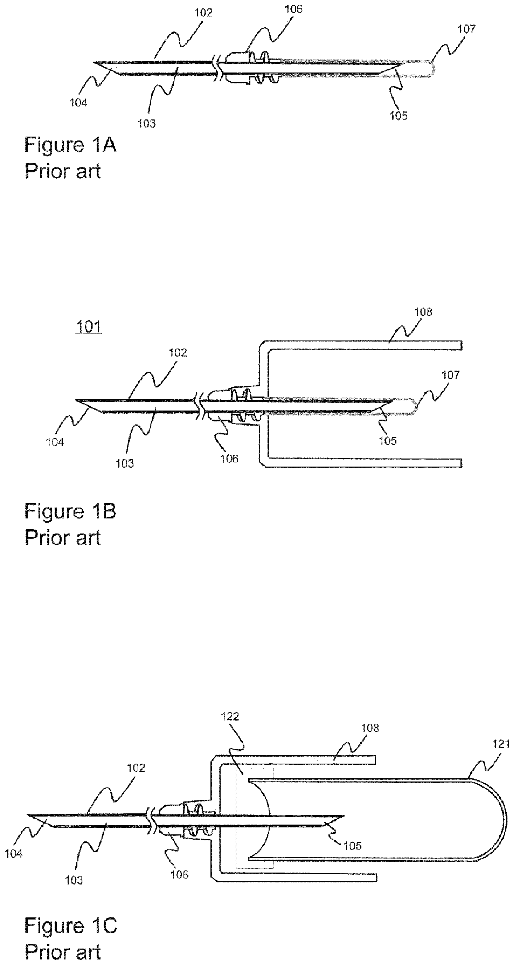

A conventional blood collection needle and a conventional blood collection needle assembly are illustrated by Figures 1A-1C (prior art). Figure 1A shows a blood collection needle **102,** which is hollow metallic needle, with a male threaded connector **106** and an elastic blood stopper **107.** Said blood collection needle **102** is thus provided with a sharp insertion end **104,** referred herein as a distal end or tip of the needle, and with a proximal end **105** through which blood flow into a blood collection vacuum tube normally occurs. Lumen of said blood collection needle is designated as **103.** Fig. 1B illustrates a conventional blood collection needle assembly **101,** which assembly comprises an above disclosed blood collection needle **102** and a tube holder **108.** Fig. 1C illustrates a conventional blood collection needle assembly **101** with a vacuum blood collection tube **121** attached thereto. Vacuum blood collection tube is sealed with a cap **122,** which is normally being tapped by the proximal end **105** of the blood collection needle **102** while connecting the blood collection tube **121** to the blood collection needle assembly **101.**

A conventional process of collecting blood samples from a blood vessel by means of conventional blood collection needle assembly **101** mainly comprises of the following steps:
- connecting a blood collection needle **102** to a tube holder **108;**
- puncturing skin with an insertion end **104** of said needle;
- performing venipuncture, i.e. penetrating the wall of a blood vessel with an insertion end **104** of said needle;
- connecting vacuum blood collection tube **121** to the blood collection needle assembly **101.**

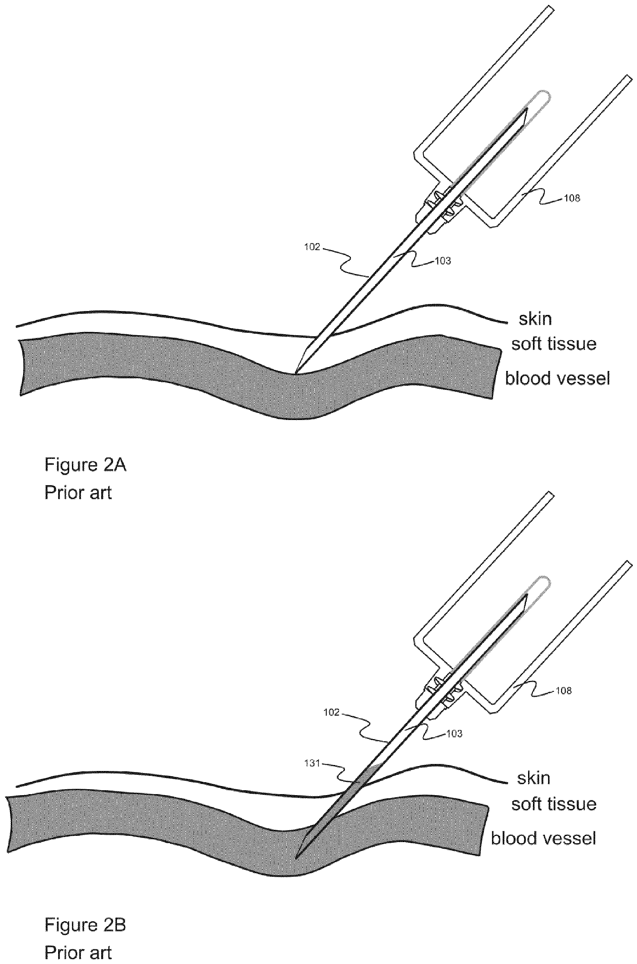

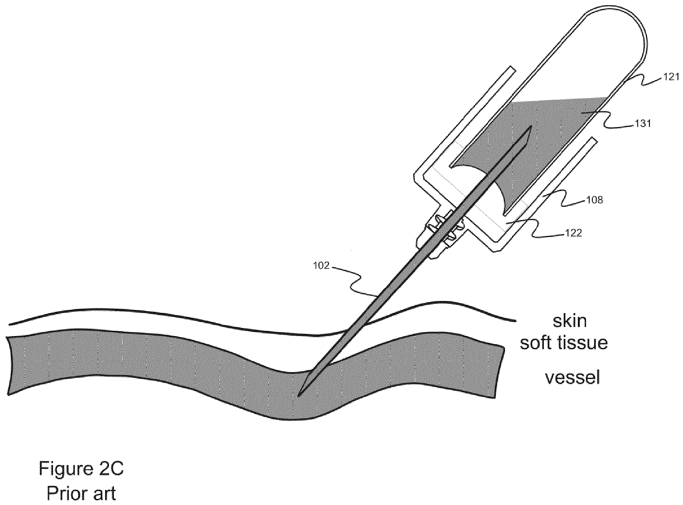

Above said process is further disclosed by an example of Figures 2A-C (prior art). Fig.2A thus illustrates an exemplary conventional blood collection needle assembly **101** at the moment of skin and tissue puncture; blood vessel is not punctured yet. Fig.2B shows the moment of venipuncture: blood flows into the lumen of the needle **102.** At the moment of venipuncture vacuum blood collection tube **121** is to be connected to the blood collection needle assembly **101** in order to collect blood flowing from a proximal end **105** of the needle **102** into sample tube **121.** Fig. 2C thus shows the moment blood flows to the blood collection tube **121,** provided that the blood vessel is punctured correctly. In conventional blood collection systems the event of blood vessel penetration may only be detected by observing a blood flow into a blood collection tube.

Blood collection needles comprising a transparent flashback chamber window are known (e.g. Vacuette Visio Plus). However, commercially available systems commonly rely to visual monitoring of blood flow into a vacuum tube. By means of conventional systems it is yet not possible to detect whether blood vessel is punctured correctly, i.e. whether the needle tip is positioned within a lumen of a blood vessel and has not punctured a blood vessel throughout. Conventional systems do not provide any additional benefits while puncturing small, thin, deep and/or damaged blood vessels, often causing medical practitioner repeat venipuncture procedure again and again thus causing damages to blood vessel walls and make patient to suffer unnecessary stress. In cases of emergency and/or performing venipuncture on kids a fast and correct insertion of the blood collection needle inside the blood vessel is of particular importance.

A blood collection needle assembly in accordance with the preferred embodiment of the present invention is configured preferably as a disposable unit comprising a rigid hollow needle with an adapter lock and a tube holder, wherein the needle incorporates at least one light guide positioned within the lumen and penetrating the tube wall of the needle via at least one aperture, said light guide is configured to receive by its proximal end light from a light source, to conduct the light throughout internal space thereof and to emit light at a distal end thereof; wherein a light wavelength is selected to be strongly absorbed by blood red cells and walls of blood vessels; and wherein an adapter lock is preferably manufactured from the material substantially non-transparent to visible light.

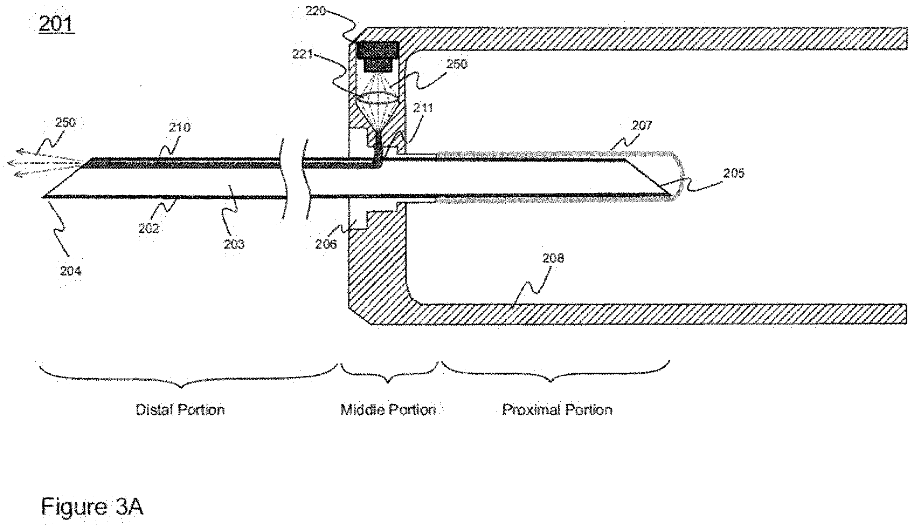

A blood collection needle assembly **201** is illustrated by Fig.3A. Blood collection needle assembly **201** of Fig.3A comprises a tubular blood collection needle **202,** which has a tube wall defining a lumen, an adapter lock **206,** an elastic blood stopper **207** and a tube holder **208.** Blood collection needle **202** in this embodiment is a rigid, tubular, hollow member, provided with a sharp distal end **204** and a proximal end **205,** and with at least one additional aperture **211** as disclosed below. Adapter lock **206** herein is embodied as a modified adaptation of a threaded connector of a conventional system, by which a tube holder may be connected to a blood collection needle. Adapter lock is arranged to keep the tube holder **208** in still pre-defined position relative to other elements of the blood collection needle assembly. Adapter lock **206,** in accordance to this embodiment, is preferably but not exclusively manufactured from substantially non transparent material, such a plastic, for example. However, in further embodiments an adapter lock may be realized as a transparent, a semi-transparent or at least a translucent part.

For clarity purposes, when referring to blood collection needle **202,** such terms as "distal portion", "middle portion" and "proximal portion" are utilized. Distal portion of the blood collection needle in reference to Fig. 3A is that tubular portion located distally from the adapter lock **206.** The portion comprising adapter lock **206** is in reference to Fig. 3A a middle portion, and, correspondingly, a portion of the blood collection needle located proximally from the adapter lock **206** is a proximal portion. An elastic blood stopper **207** is thus arranged at a proximal portion.

Along a portion of the lumen of the blood collection needle **202** a light guide **210** extends. Said light guide **210** may be a single or multicore optical fiber, for example, or any other suitable means for transmitting visible light. One end of the light guide **210** extends through the tube wall of the blood collection needle **202,** thus forming a light entry aperture **211** in the tube wall that is located substantially in the middle portion of the needle **202** between the distal and the proximal ends thereof and leveled with the adapter lock **206.** The light guide **210** penetrates also the adapter lock **206.** For clarity purposes aperture **211** will be further disclosed as to penetrate the tube wall of the needle **202** and the adapter lock at once unless otherwise stated. The tip of the light guide is arranged to terminate within the lumen **203** of the blood collection needle **202** in close proximity to its distal end **204** and/or inside a blood entry aperture. The light guide **210** is configured to receive by its proximal end visible light from a light source **220** and to emit the visible light at the distal end **204** of the needle **202.** Therefore light guide **210** is implemented as a curved element, wherein the curve angle is between 60 and 175°, preferably between 90° and 170°. In other words deviation of a light guide from the straight line will be 15 degrees at a light guide angle of 165 degrees. Light source **220** is preferably implemented as a laser source comprising at least one laser; however, any other technically suitable illumination means, such as LEDs or the like, are possible. Visible light from light source **220** is preferably converged by a light converging means **221,** such as lens, for example. The system may be implemented also in an absence of lens with direct light input from the light source **220** into the light guide **210.** Visible light (dotted line) is thus converged by converging means **221** and is further received by the light guide **210** as described above. In accordance with the preferred embodiment, the light source **220** is incorporated within the front wall of the tube holder **208** and is substantially aligned with the light entry aperture **211.** Positioning the light source **220** in side walls of the tube holder **208** is also possible, whether technically appropriate.

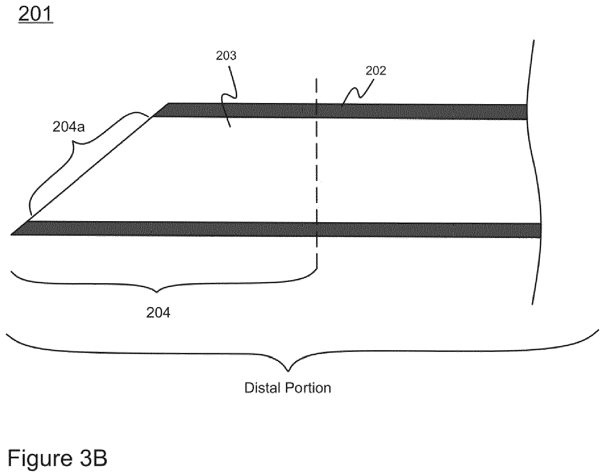

Fig. 3B schematically illustrates a distal portion of a blood collection needle of a blood collection needle assembly of the invention. The distal portion of a blood collection needle comprises a distal end **204** that generally corresponds to that portion of a needle that enters a blood vessel (vein). Length of the distal end **204** may vary depending on an individual, for example 3-5 mm. The distal portion comprises a blood entry aperture **204a.**

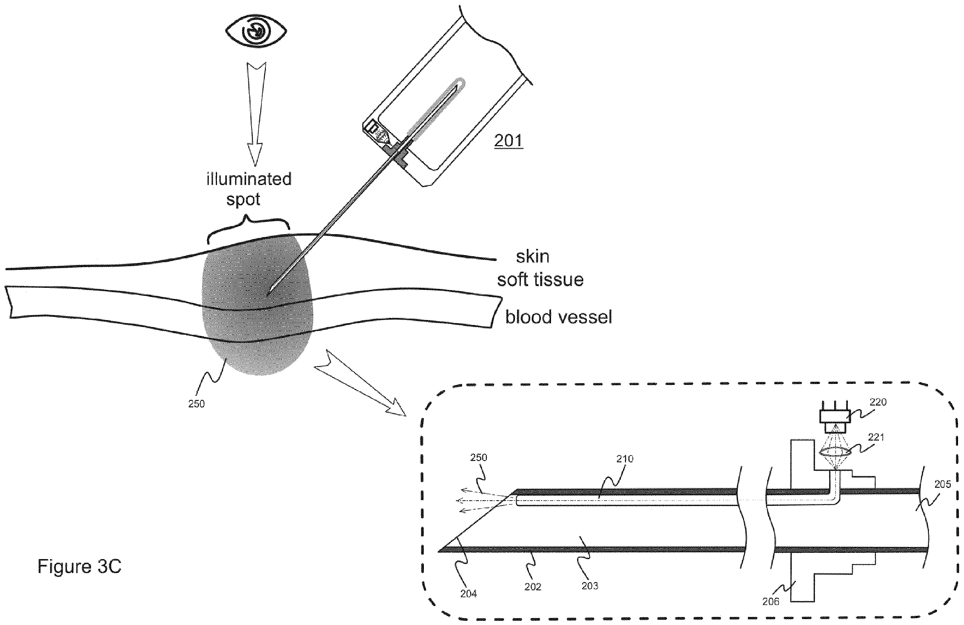

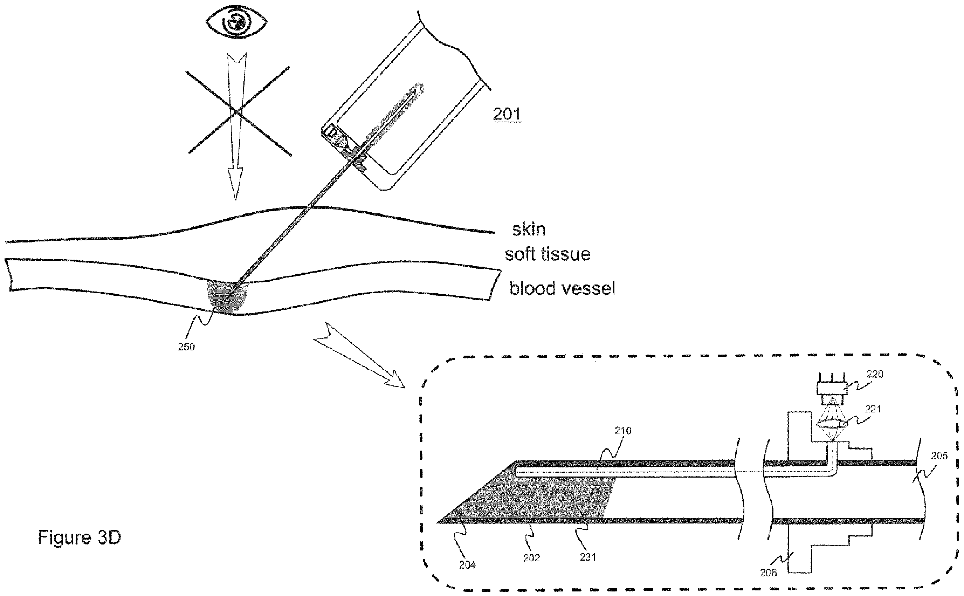

Figures 3C and 3D in turn illustrate the general procedure of blood sample collection by means of assembly **201.** Visible light **250** emitted by light source **220** is further focused by converging means (lens) **221** and directed into the light guide **210.** Light travels through the light guide **210** inside the lumen **203** of the needle until it is emitted at a distal end **204** thereof, where it may be easily detected by an outside observer (represented by eye pictogram). In accordance with the preferred embodiment of the invention, the light source **220** is adjusted to emit visible light of at least one color and/or wavelength. One of such wavelengths, being strongly absorbed by blood, in particular by red blood cells, and/or by the walls of blood vessels (such as veins and arteries), but being relatively weakly absorbed by skin, fat and other surrounding tissues, may be in the green and/or yellow waveband. More specifically, at least 40% of light contributing to the total light intensity emitted from the distal end of the light guide **210** may have a wavelength between 510 nm and 600 nm. Preferably at least 30% of light, contributing to the total intensity emitted from the distal end of the light guide **210** may have a wavelength selected from one of the following ranges: between 510 nm and 550 nm, or 530 nm and 570 nm, or 560 nm and 600 nm. Other examples of suitable wavelengths include orange and/or red wavebands. Then the light is scattered within tissues to a great extent and is able to enhance the contrast between blood vessels and surrounding tissues for external observer.

In order to reach a blood vessel, blood collection needle **202** has to penetrate tissues with different light scattering properties. Referring now to Fig. 3B, when the tip of the needle of the catheter needle assembly **201** had already penetrated skin and tissues but has not yet entered a blood vessel, light **250** illuminates surrounding tissues, being partly reflected and scattered therefrom. Correspondingly, an illuminated spot (Fig. 3B) may be observed on the skin surface, and a clinician may follow the movement of the needle **202** inside the skin to be aware of an exact position of the needle relative to a blood vessel. As soon as needle tip enters a blood vessel (Fig. 3C), light is absorbed by blood and/or the walls of a blood vessel, and correspondingly light spot **250** is no longer visible on skin surface from outside. Since the penetration of a blood vessel wall by the needle tip is a very rapid process, optical properties of needle tip surrounding environment change momentarily from relatively low light absorption level to very high light absorption level. As can be seen from figures 3B and 3C, light spot **250** becomes 'trapped' inside the blood vessel upon needle tip punctures the wall of a blood vessel and enters the lumen thereof. As a result, an outside observer may easily visually detect the moment when light, otherwise visible on skin, disappears; which event is indicative of a successful venipuncture. The other events, happening upon needle tip penetration into blood vessel, may comprise rapid weakening of light, rapid change of light color and rapid change of light color along with rapid weakening. These events may be observed by eye and depend on the chosen light wavelength.

Above described phenomena may be observed upon selecting light of specific wavelengths and specific optical powers. An event of absorption of light by blood upon successful introduction of the needle into a blood vessel may be registered by eye. Particularly if non-visible light is emitted by the light source **220,** a detection system of any suitable kind may be used to detect these phenomena. If blood vessels are small in diameter and/or have thin walls, minor part of light, delivered into a blood vessel from the tip of the blood collection needle, may escape and be scattered in the surrounding tissues, thus remaining or becoming again visible for a clinician. In this case light might not disappear from view completely, but become instantly weaker upon penetration inside a blood vessel by the tip of the blood collection needle. Instant change of light color under skin may be indicative of a successful venipuncture when light of more than one wavelength is delivered into the light guide.

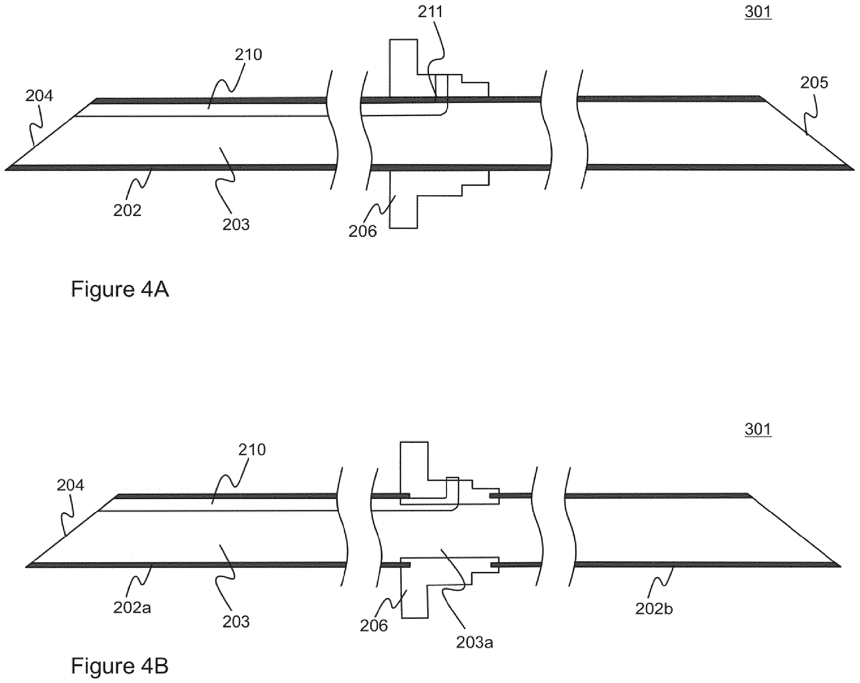

On Figures 4-8 and 10 the blood collection needle with the adapter lock is shown, but the tube holder is omitted from said drawings for clarity purposes. The configuration consisting of the blood collection needle **202** and the adapter lock **206** will thus be designated by reference numeral **301.**

Figure 4B discloses a blood collection needle of the blood collection needle assembly in accordance with another embodiment of the invention, as compared to the blood collection needle of the blood collection needle assembly in accordance with the above disclosed preferred embodiment (Fig. 4A). The blood collection needle assembly may thus comprise the blood collection needle built up of two separate hollow tubular units **202a** and **202b** arranged coaxially to each other and joined together by the adapter lock **206** (Fig. 4B). Manufacturing expenses for said embodiment are smaller, since there is no need to drill a hole in the metallic core of the blood collection needle. Unit **202a** is thus distal unit of the blood collection needle, and unit **202b** is proximal unit of the blood collection needle. Units **202a** and **202b** of the blood collection needle are thus sealed with an above disclosed plastic adapter lock **206,** the wall thereof thus incorporates an aperture for the proximal end of the light guide **210.** Units **202a** and **202b** may be melted into the plastic adapter lock **206,** for example, or otherwise incorporated therein by any technically suitable manufacturing process.

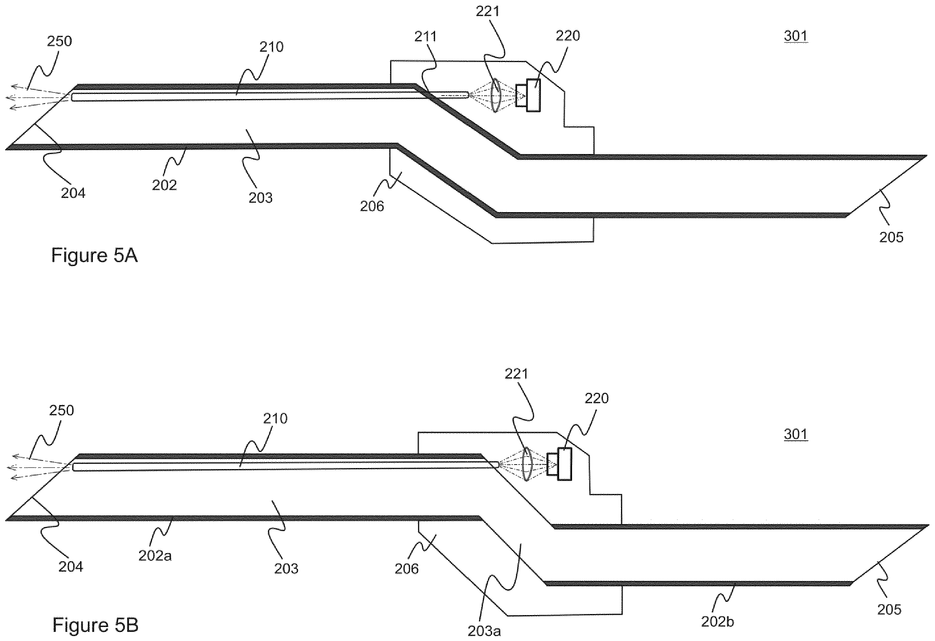

Figures 5A and 5B illustrate other embodiments of the blood collection needle **202.** Figure 5A thus illustrates a one-piece blood collection needle of the blood collection needle assembly, which needle is provided with a slanted middle section, so, that distal and proximal ends of said blood collection needle have different axes. The term "slanted middle section" refers in this disclosure to a configuration **301,** comprising a blood collection needle adapted to bend twice in substantially middle section thereof, thus creating a structure with two straight parallel sections, distal and proximal herein, and one slanted section, a middle section herein. An adapter lock is preferably positioned over the slanted middle section. Because the length of said middle section is very small and so is a bend angle, the user may not detect a difference between a straight needle configuration and a needle configuration with slanted middle section. The benefit of such an arrangement is that the need to use a light guide having a bent portion is eliminated. The light guide **210** thus penetrates the wall of the needle **202** at slanted middle section through an aperture **211** without adapting a curved shape. Slanted middle section of the needle **202** is preferably integrated into an adapter lock **206.** Light source **220** and lens **221** are positioned in such a way, to focus light at a proximal end of the light guide **210.**

The arrangement of Figure 5B is substantially the same as for Fig.5A, except that, the blood collection needle illustrated therein is built up of two separate units **202a** and **202b,** arranged parallel to each other, in accordance with definition recorded elsewhere in this document, and joined together by the adapter lock. Technically the embodiment of Fig.5B is close to that of Fig.4B, however, units **202a** and **202b** of Fig.5B are shifted in relation to each other. Space indicated by **212** (Fig.5B) is thus provided only by the walls of the adapter lock **206.** Respectively, the need in an aperture **211** in the needle **202** wall is eliminated. Light is directed to the proximal end of the light guide **210** through a substantially non-transparent adapter lock. The shape of the adapter lock **206** at Figures 5A and 5B is exemplary and it may be adjusted in accordance with technical requirements for the blood collection needle assembly of the invention.

Arrangement represented by Figs. 5A and 5B are exemplary and are not intended to limit the invention. Units **202a** and **202b** may be positioned in different positions and/or shifted by various angles in relation to each other.

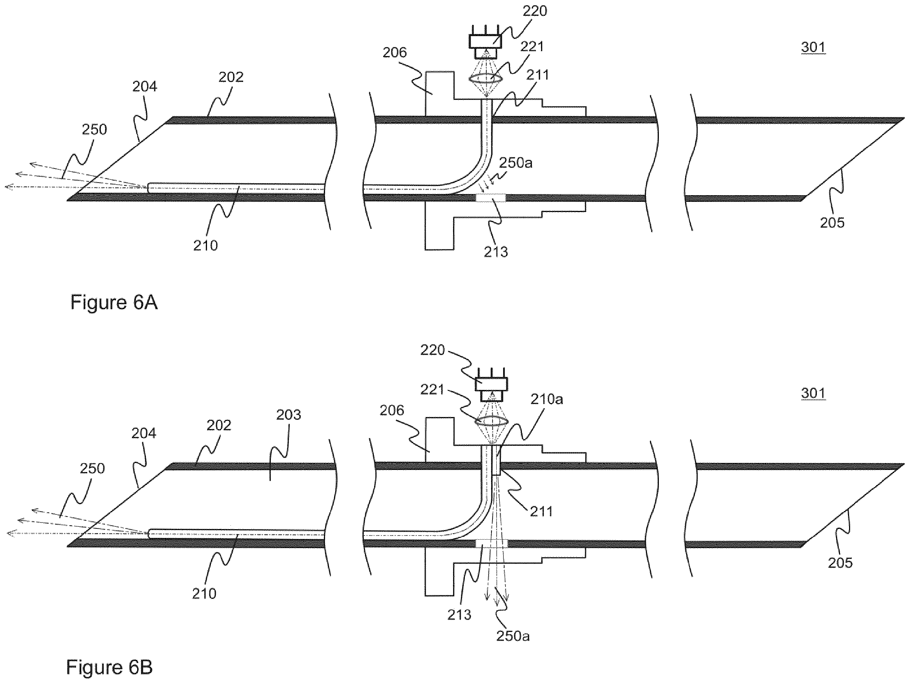

By an example of Fig. 6A another embodiment of the invention is disclosed, wherein the blood collection needle of the blood collection needle assembly comprises an additional aperture **213** in the wall thereof. Said additional aperture **213** may be referred for clarity purposes as a "light exit window" in the needle wall, said window is positioned substantially opposite to the aperture **211** as well as to curve adapted by the light guide **210.** The term "window" reveals best the purpose of said additional aperture **213,** which serves substantially as means for light **250a** to pass through the window and to illuminate said adapter lock from inside, as disclosed further. Rays of incident light **250a** may thus be detected visually through the window **213** as long as the needle has not yet penetrated the blood vessel. Rays of incident light **250a** passing through the window **213** are indicated by small arrows (Fig. 6A).

Fig. 6B illustrates still another embodiment of the invention, wherein the blood collection needle **202** of the blood collection needle assembly may comprise more than one light guide in the needle's lumen. Figure 6B thus illustrates the blood collection needle with two light guides, **210** and **210a,** the proximal ends thereof penetrate the wall of the metallic needle **202** through the aperture **211.** The light exit window **213** is arranged substantially opposite both light guides as well as the curve adapted by the light guide **210** in a manner disclosed above and illustrated by Fig.6A. The first light guide **210** herein is arranged in accordance with the preferred embodiment of the invention, and the second light guide **210a,** being significantly shorter, is arranged with its distal end to point the lumen **203** of the needle **202** and with its proximal end to penetrate the wall of the needle **202** through an aperture **211.** Light guides **210** and **210a** are situated in close proximity to each other. The advantage of a second light guide is attaining an additional illumination further emitted through the light exit window **213** in a manner disclosed below.

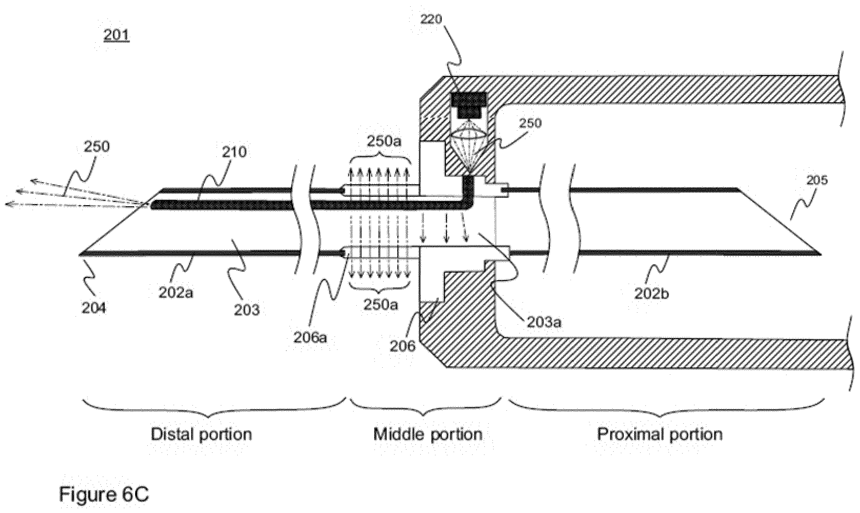

Referring now to Fig. 6C, the blood collection needle assembly **201** is presented, comprising an adapter lock **206** provided with a transparent elongated distal section **206a.** For clarity purposes the elongated adapter section **206a** is referred to as a part of the middle portion of the blood collection needle. The distal and proximal portions thus correspond to the distal unit **202a** and the proximal **202b** of the blood collection needle, respectively. Although the adapter **206** may be as well transparent for visible light, as semi- or non-transparent, the section **206a** is transparent for visible light. Inner volume of section **206a** forms a flashback chamber, where blood can be visually observed upon successful venipuncture. Flashback chamber thus forms a continuous path for blood to flow from the vein to the blood collection tube via the lumen **203** of the blood collection needle. A portion of a light guide **210** may be visually observed within flashback chamber. Light guide **210** may thus be observed better when the light source **220** is switched on.

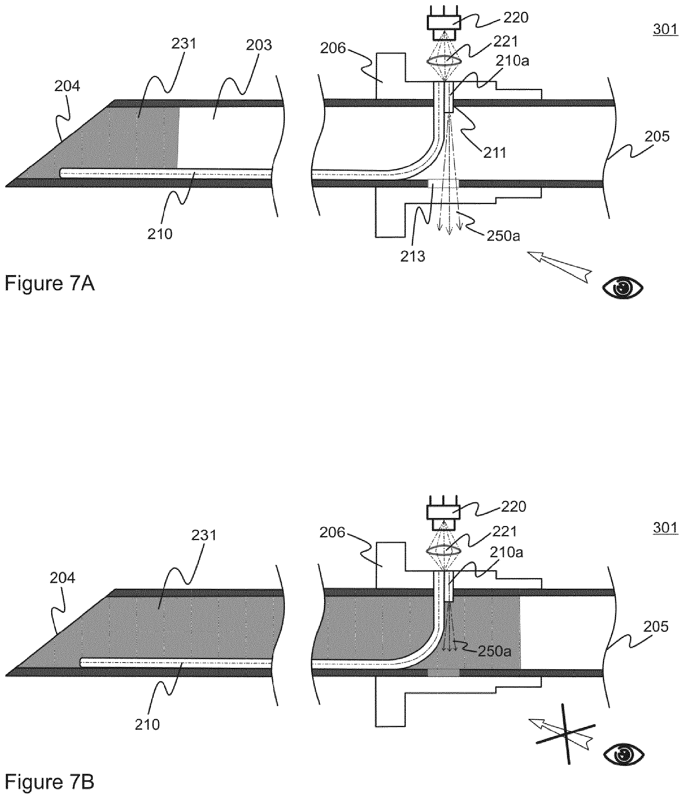

Figures 6B, 7A and 7B illustrate same technical solution for the blood collection needle comprising two light guides. Figs. 7A and 7B illustrate an exemplary process of blood flow into the lumen of said needle. Fig. 6B may be much referred as a complementary to Fig. 3B, wherein the light source is switched on, but the blood collection needle has not entered blood vessel yet. Light is emitted from the distal end **204** by the first light guide **210** and through the window **213** by the second light guide **210a.** Light passing window **213** makes the whole adapter lock **206** illuminate, thus creating an impression of glow. The adapter lock **206** is thus preferably manufactured from substantially semi-transparent material. Fig. 7A may be referred to as well as a complementary to Fig. 3C, wherein the light source is switched on, but the needle **202** had already penetrated blood vessel and some blood **231** had already flown inside the lumen **203** of the needle **202.** In accordance with the above disclosed phenomena of light of certain wavelengths absorption by red blood cells, light is not visible anymore at a distal end **204.** However, window **213** still passes incident rays from the second light guide **210a** that makes the semitransparent plastic adapter **206** still glow. Fig. 7B illustrates a subsequent moment, when blood **231** flows further to the lumen of the needle **202** and occupies the whole diameter of the lumen. Light **250** emitted by the second light guide **210a** is now blocked as well, and the plastic semi-transparent adapter lock **206** is not illuminated from the inside anymore. Light **250** is thus blocked when window **213,** light guides **210, 210a** or all these elements are obstructed by blood.

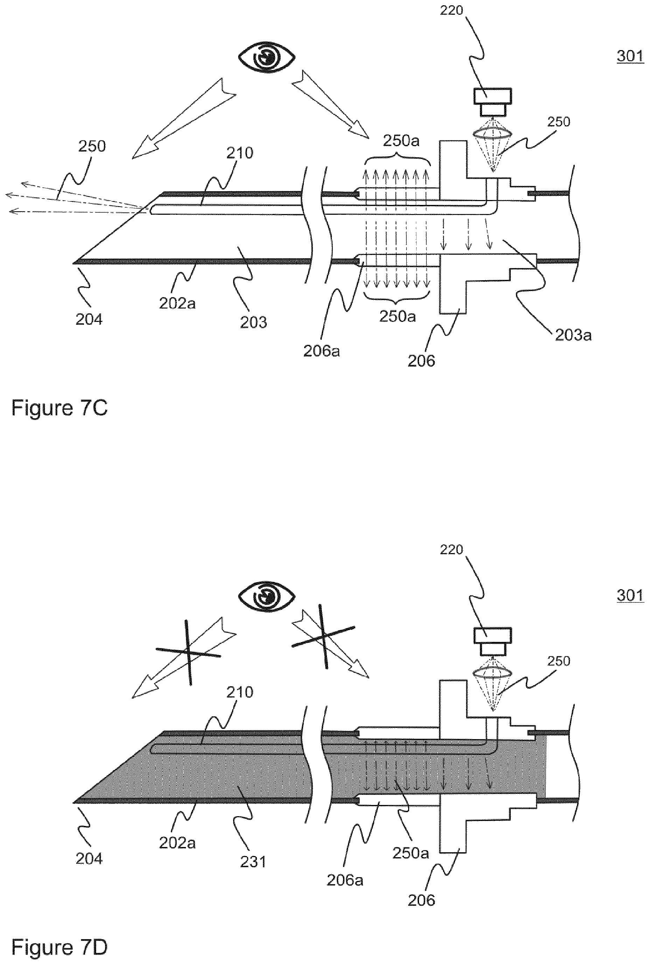

Figs. 6C, 7C and 7D illustrate illumination events already described in regards to Figs. 7A and 7B, but related to the blood collection needle provided with an adapter with a flashback chamber (Fig. 6C). As blood enters flashback chamber, light otherwise visible to an observer, disappears from the sight thereof (Fig. 7D), thus providing an additional evidence of blood collection needle entering the vein. An observer can in addition see blood flowing into the blood collection tube by eye.

A clinician may thus be aware of 1) the moment of blood vessel penetration (Fig. 7A) and of 2) the correctness of blood vessel penetration indicated by unobstructed blood flow into the lumen of the needle (Fig. 7B). First observation is made on the basis of light disappearance from the distal end **204** of the blood collection needle, and the second observation is made by monitoring an illuminated state of the adapter lock **206.** When blood flow blocks incident rays of light **250** from penetrating the window **213,** adapter lock is not anymore illuminated (Fig. 7B).

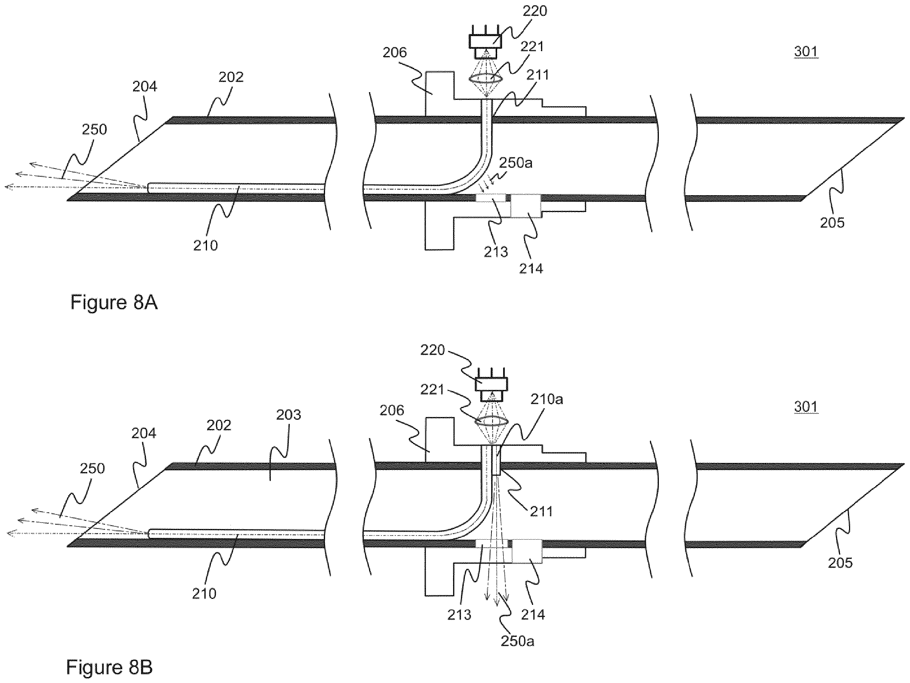

The blood collection needle in accordance with above described embodiments (Figs. 6A, 6B, 7A, 7B) may be further enhanced with a venting mechanism, which would allow air to escape from the proximal section of the blood collection needle, but would prevent flow of blood from the lumen of the needle. The blood collection needle with an adapter lock configuration **301,** comprising such venting mechanism, is illustrated by Figs. 8A and 8B. Figure 8A shows a configuration **301** with one light guide **210** and Figure 8B shows a configuration **301** with two light guides **210** and **210a.** Venting mechanism is preferably implemented as an additional aperture **214** in the wall of the blood collection needle **202** sealed with a porous, air-permeable filter-like material, located proximal to optically transparent window **213.** Alternatively vent **214** may be implemented as a part of an adapter lock **206.** The purpose of venting mechanism **214** herein is to allow air outflow, but to prevent blood outflow to ambient surroundings.

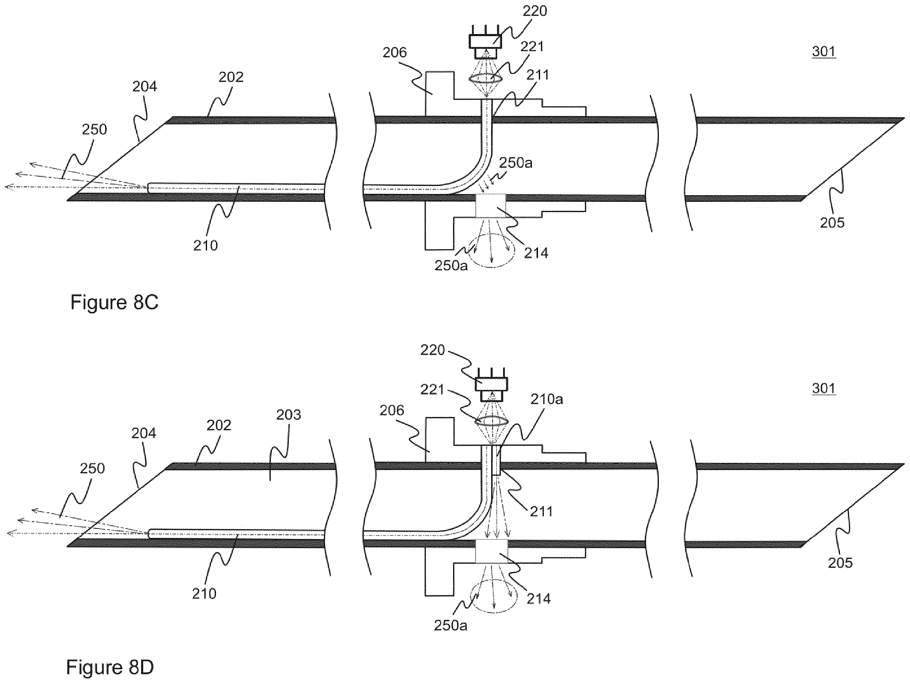

Figures 8C and 8D illustrate the configuration **301** similar to that of Figs. 8A and 8B, but comprising only venting mechanism **214** at the place of the additional aperture **213.** Fig. 8C thus illustrates a configuration **301** with one light guide **210** and Figure 8D illustrates a configuration **301** with two light guides **210** and **210a.** In this configuration vent **214** is preferably implemented as an aperture in the wall of the blood collection needle **202** sealed with a porous, air-permeable filter-like material, which material is also substantially semi-transparent to light. In comparison to the solution of Figs. 8A-B, wherein vent **214** may be implemented as a non-transparent member, vent **214** of Figs. 8C-D is adapted to replace window **213** and to provide an additional aperture for light **250** to pass through the wall of the needle, in accordance with disclosed above, combined with ventilation means.

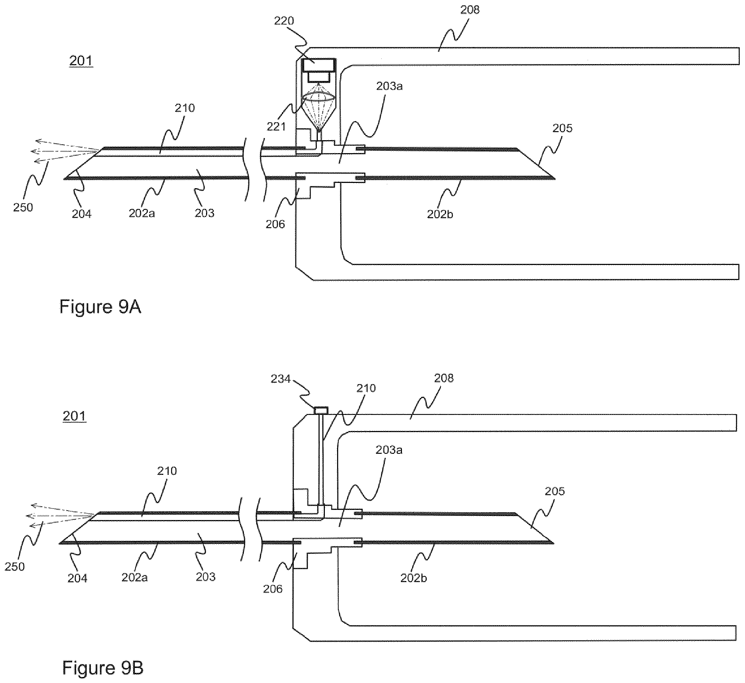

Figures 9A-D illustrate further embodiments of the invention, concerning the arrangement of electronic and optical components within the tube holder **208.** Fig. 9A thus illustrates the blood collection needle assembly of the invention substantially same to that of Fig. 4B, wherein the blood collection needle is comprised of two units **202a** and **202b** arranged coaxially to each other, in accordance with definition disclosed elsewhere in this document, and joined together by the adapter lock, as disclosed above. Blood collection needle assembly **201** of the invention comprises a tube holder with thicker walls as comparing to that of prior art systems, in order to incorporate electronic and optical components. This especially applies to the thickness of a front wall of the tube holder, directly connectable to the adapter lock **206** of the blood collection needle **202.** As illustrated by Fig. 9A (and 3A), the tube holder **208** comprises a light source **220,** such as laser, LED or the like, and light converging means **221** as an optical element, such as lens, for example. Tube holder may also incorporate the part of a light guide **210,** conventional or rechargeable battery for a light source (not shown) and an electronic module in order to operate a light radiation source (not shown). The tube holder may thus comprise a suitable sort of switch for switching a light source on and off.

The light source **220** along with light converging means **221** may thus be arranged directly within the front wall of the tube holder **208.** The light guide **210** is thus arranged to penetrate the wall of the adapter lock **206** and to receive light from the light source **220.** The blood collection needle may be either one-piece, as represented on Fig. 3A, or comprise two units **202a** and **202b** joined together by the adapter lock **206** in above disclosed manner, as represented by Fig. 9A.

In accordance with some embodiment, the blood collection needle assembly **201** may comprise an internal light guide **210** incorporated within the front wall of the tube holder and a coupling connector **234** (Fig. 9B), wherein said coupling connector is adjusted to receive an external light guide or an external replaceable source of light (not shown). Light guide **210** herein penetrates the wall of the adapter lock **206** and further continues through the front wall of the tube holder **208** along the cross-section terminating at coupling connector **234.** Said coupling connector **234** may be built within the tube holder **208** or on an outer wall thereof (as illustrated by Fig. 9B). The arrangement thus disclosed may reduce manufacturing expenses, while disposable assembly **201** of Fig. 9B may be directly connected to a permanent light source, for example, by means of multiple use external light guide. The blood collection needle may be either one-piece, or comprise two units **202a** and **202b** joined together by the adapter lock **206** in above disclosed manner (Fig. 9B).

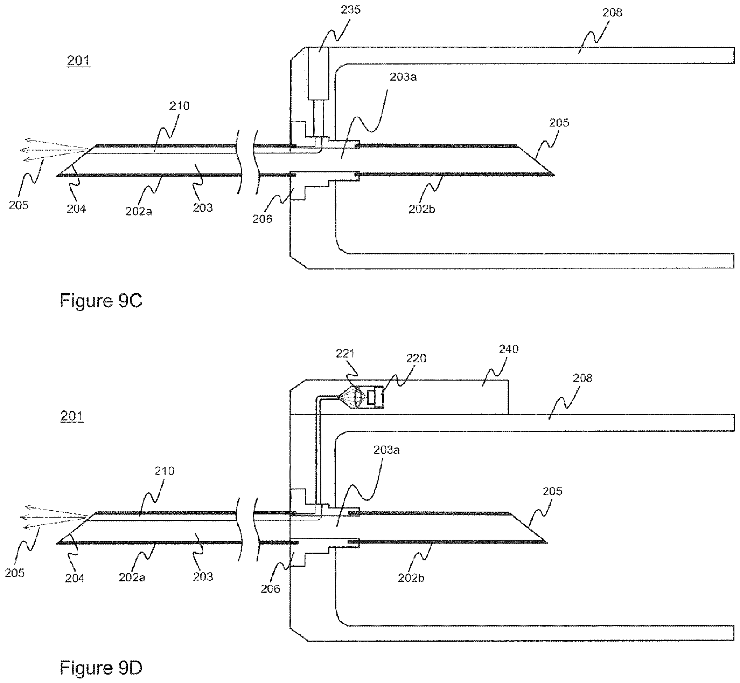

In accordance with some other embodiment, the blood collection needle assembly **201** may comprise an internal light guide **210** arranged to penetrate the adapter lock **206,** and a coupling mechanism in the form of a hollow element **235** incorporated within the front wall of the tube holder in order to receive an above mentioned external light guide (Fig. 9C, external light guide not shown). The blood collection needle may be either one-piece, or comprise two units **202a** and **202b** joined together by the adapter lock **206** in above disclosed manner (Fig. 9C).

In accordance with some other embodiment of the invention the blood collection needle assembly **201** may comprise an internal light guide **210** incorporated within the front wall of the tube holder and the light source **220** with light converging means **221** arranged within a separate unit **240** releasably or non-releasably fixed to the tube holder **208** (Fig. 9D). The arrangement is otherwise substantially similar as disclosed with regards to Fig. 9A, except that the assembly **201** comprises an additional separate unit **240.** The blood collection needle may be either one-piece, or comprise two units **202a** and **202b** joined together by the adapter lock **206** in above disclosed manner (Fig. 9D).

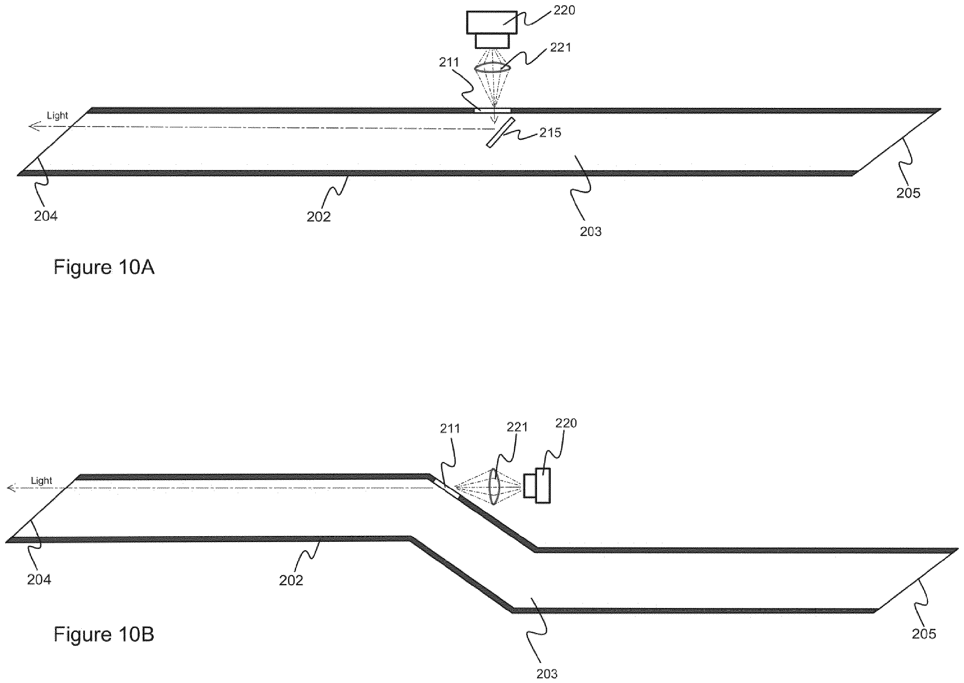

In accordance with further embodiment of the invention the light guide **210** may be omitted from the blood collection needle assembly, as illustrated by Figs.10A-C. Instead, reflective inner surface of the needle **202** may operate as a light transmitting surface. Light beam is represented by dashed line inside the needle **202.** A one-piece blood collection needle with a light guide omitted is thus shown on Fig.10A. Adapter lock is not shown. Light, focused by lens **221,** passes an aperture **211** and hits an additional optical element **215,** such as mirror, for example, is reflected therefrom to be transmitted along the lumen **203** of the needle **202** and to be emitted at a distal end **204** thereof.

Fig. 10B represents a solid blood collection needle with a slanted middle section, as disclosed above; the light guide is omitted therefrom (adapter lock is not shown). In similar manner, light focused by lens **221** passes an aperture **211** and continues along the reflective inner surface of the needle **202** to be emitted at a distal end **204** thereof. No optical element, such as mirror, is required in this case.

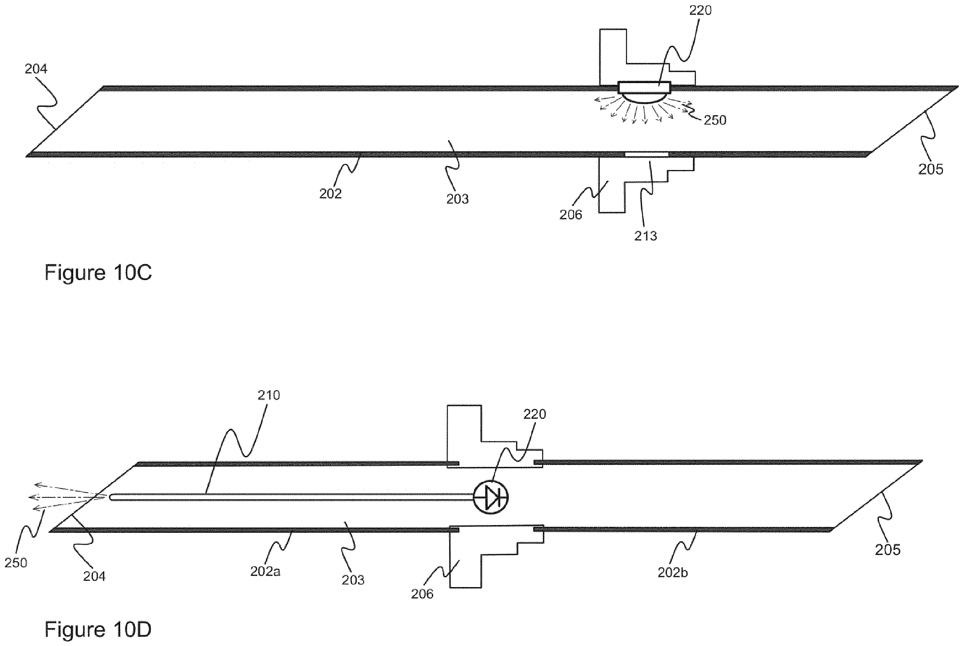

Fig. 10C illustrates another substantially additional embodiment, wherein the light source **220** is arranged to be incorporated directly into the wall of the blood collection needle **202** or into a transparent window **213.** In the latter case widow **213** would act as a light guide to deliver light into the lumen of the blood collection needle. The window **213** herein enables a double-detection performance, wherein light, emitted by the light source **220** may be visible both at a distal end **204** and through the substantially transparent or semitransparent walls of the adapter lock **206.**

Fig. 10D represents another embodiment of the invention, wherein the light source **220** may be arranged within the lumen **203** of the blood collection needle, comprised of two units **202a** and **202b,** joined together by the adapter lock **206.** Said embodiment may be also applied for a one-piece blood collection needle, comprising a window **213,** for example. The arrangement thus disclosed enables a double-detection performance, wherein light, emitted by the light source **220** may be visible both at a distal end **204** and through the substantially transparent or semitransparent walls of the adapter lock **206.** Light, emitted by the light source **220** may either be transferred along the light guide **210** (Fig. 10D), or alternatively the blood collection needle with an omitted light guide may be implemented, in accordance with embodiments disclosed above.

In further, substantially additional embodiment of the invention, a venting mechanism for communication between the lumen **203** of the blood collection needle **202** and ambient surroundings is provided, wherein said venting mechanism permits an air outflow from the lumen of said needle, however, substantially preventing a fluid outflow from said needle assembly to ambient surroundings. Said venting mechanism may be preferably implemented as an above disclosed additional aperture **214** manufactured from porous, white, translucent material. Said venting mechanism may be arranged anywhere within the adapter **206,** preferably, but not exclusively, proximal or at the same level to aperture **211,** therethrough light is allowed to pass into the lumen of the blood collection needle. Said venting mechanism may further be implemented as a venting shaft, incorporated into an additional aperture, arranged in the wall of the blood collection needle **202,** wherein said venting shaft is implemented to connect the lumen **203** of the blood collection needle **202** and ambient surroundings. Said venting shaft is sealed with an air-permeable member preferably manufactured from porous filter material in order to pass out air being displaced by fluid, which material is preferably adapted to glow while illuminated.

The adapter lock **206** and the tube holder **208** additionally may comprise an additional safety feature implemented as an ON/OFF switch to control operation of the light source **220.** Said switch may be any switch technically suitable for purposes of the invention. Switch can be implemented to be activated upon mounting the blood collection needle onto the tube holder, and inactivated upon unmounting the blood collection needle from the tube holder. The blood collection needle assembly may be provided with additional switches for regulating brightness, color, intensity and the like of the light source(s).

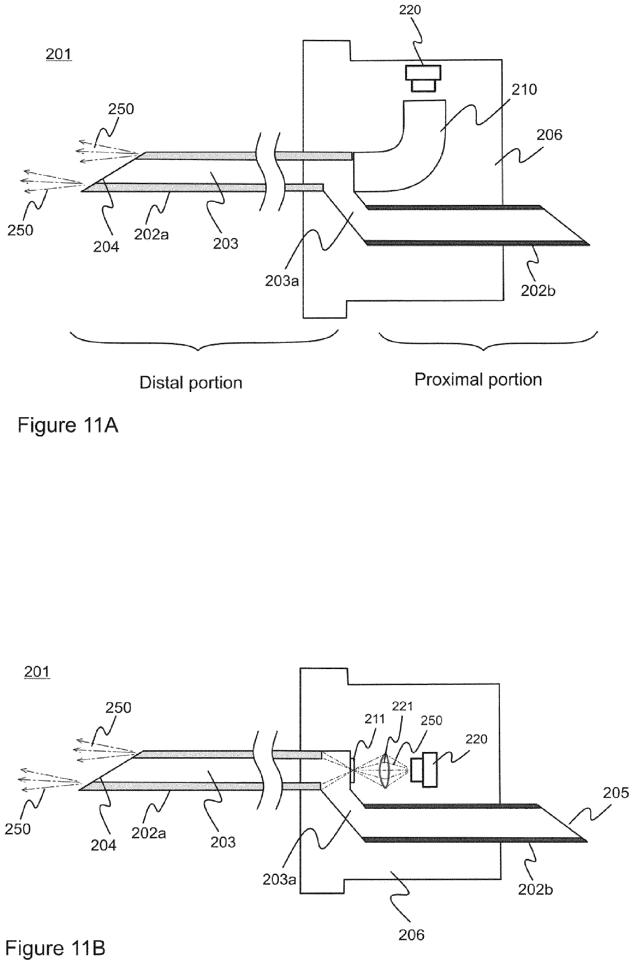

Figs. 11A and 11B illustrate exemplary embodiments of the invention, wherein the distal portion of the blood collection needle is manufactured from a material other than metal.

Fig. 11A shows an exemplary arrangement of the blood collection needle assembly (tube holder **208** is not shown), wherein the light guide **210** forms, at least partially, an integral unit with the distal portion of the hollow blood collection needle. Fig. 11A illustrates the distal portion of the blood collection needle **202a** connected to a light guide **210.** Light guide **210,** represented by an optical fiber, for example, does not extend to the lumen of the distal portion of the blood collection needle **202a,** in accordance to this embodiment, but the distal portion of the blood collection needle **202a** is realized so, to be capable of acting as a light guide itself. Such implementation may be realized by means of manufacturing the distal portion of the blood collection needle from a material, other than metal, for example. Additional elements may be implemented hereto to prevent light losses through a distal portion of the blood collection needle, such as, a surface coating, an additional metallic pipe or a catheter tubing-like part disposed onto or close to the outer surface of the distal portion of the blood collection needle, in accordance to this embodiment. The proximal portion of the blood collection needle **202b** illustrated by Fig. 11A is preferably, but not exclusively provided as a component manufactured from metal. Materials other than metals are not thus excluded.

Fig. 11B illustrates further embodiment of the invention, wherein the blood collection needle comprises of two separate tubular units **202a** and **202b** and the distal unit **202a** is manufactured from the material alternative to metal and possessing good light conducting properties. Mentioned material may be plastic-based, for example. Distal tubular unit **202a** of the blood collection needle of Fig. 11B may be manufactured from a transparent plastic so, that light can be conducted directly by the walls of the distal tubular unit, not only by the lumen **203.** This is the case, when no additional light guiding element(s) within the lumen **203** are required because the whole unit **202a** serves as light guiding element. Unit **202a** may be provided with a cladding layer either on an outer surface or on both inner and outer surfaces thereof, in order to minimize losses due to light exiting directly from the walls of the distal tubular member **202a,** acting herein as a light guiding element. Preferably the external surfaces of unit **202a** are more due to be produced with a cladding layer, so as light leaving said unit **202a** (acting as a light guide, herein) towards the lumen **203** thereof will not be lost. Also described in the present specification is a method for intracutaneous localization of the blood vessels during blood collection, for detection an exact moment of the intravascular penetration and for ensuring a correct position of the blood collection needle inside blood vessel is provided in accordance with aforesaid embodiments, said method comprises at least several of the following steps:
a. obtaining the blood collection needle assembly **201;**
b. activating an optical radiation source **220,** provided with the blood collection needle assembly **201,** wherein said optical radiation source is adapted to emit optical radiation of such a wavelength(s), that is strongly absorbed by blood red cells and/or walls of blood vessels and is reflected and/or scattered from blood vessel surrounding tissues, wherein optical radiation of chosen wavelength can enhance contrast between blood vessels and/or surrounding tissues, thus allowing improved blood vessel visualization for a clinician;
c. puncturing the skin with the blood collection needle **202** of the blood collection assembly **201;**
d. localizing a blood vessel position intracutaneously by monitoring illumination events at the distal end **204** of the blood collection needle **202** and/or by traditional means;
e. detecting a moment of blood vessel penetration by the blood collection needle **202** by monitoring illumination events underneath the skin, said events comprise at least one of the following: instant disappearance of illumination from the distal end **204** of the blood collection needle **202,** rapid illumination fading, rapid change in light color or rapid illumination fading along with rapid change in light color, wherein said events take place upon entering the lumen of the blood vessel with the tip of said needle and wherein said events are dependent on the optical radiation wavelength being utilized;
f. ensuring a correct position of the blood collection needle **202** inside the lumen of a blood vessel by monitoring illumination events at the adapter lock **206** element, wherein an unobstructed blood flow into the lumen **203** of the blood collection needle causes illumination at the adapter lock **206** and/or window **213** to disappear at once.
Further is described a method for intracutaneous localization of the blood vessels during blood collection, for detection an exact moment of the intravascular penetration and for ensuring a correct position of the blood collection needle inside blood vessel is provided by means of observation of illumination events in the flashback chamber in accordance with some aforesaid embodiments, said method comprises at least several of the following steps:
a. obtaining the blood collection needle assembly **201** provided with the adapter lock equipped with a transparent elongated distal section **206a** which inner volume forms a flashback chamber;
b. activating an optical radiation source **220,** provided with the blood collection needle assembly **201,** wherein said optical radiation source is adapted to emit optical radiation of such a wavelength(s), that is strongly absorbed by blood red cells and/or walls of blood vessels and is reflected and/or scattered from blood vessel surrounding tissues, wherein optical radiation of chosen wavelength can enhance contrast between blood vessels and/or surrounding tissues, thus allowing improved blood vessel visualization for a clinician;
c. puncturing the skin with the blood collection needle **202** of the blood collection assembly **201;**
d. localizing a blood vessel position intracutaneously by monitoring illumination events at the distal end **204** of the blood collection needle **202** and/or by traditional means;
e. detecting a moment of blood vessel penetration by the blood collection needle **202** by monitoring illumination events underneath the skin, said events comprise at least one of the following: instant disappearance of illumination from the distal end **204** of the blood collection needle **202,** rapid illumination fading, rapid change in light color or rapid illumination fading along with rapid change in light color, wherein said events take place upon entering the lumen of the blood vessel with the tip of said needle and wherein said events are dependent on the optical radiation wavelength being utilized;
f. ensuring a correct position of the blood collection needle **202** inside the lumen of a blood vessel by monitoring illumination events at the transparent elongated distal section **206a** of the adapter lock, wherein an unobstructed blood flow into the lumen **203** of the blood collection needle causes illumination at section **206a** to disappear at once, and wherein blood flow is visually observable.
Yet further is described a method for obtaining a blood sample by means of the blood collection needle assembly **201** is provided in accordance with aforesaid embodiments, said method comprises:
a. identifying an appropriate blood vessel for performing venipuncture and preparing the patient skin for obtaining a blood sample;
b. applying an arm tourniquet;
c. obtaining the blood collection needle assembly **201;**
d. activating an optical radiation source **220,** provided with the blood collection needle assembly **201,** wherein said optical radiation source is adapted to emit optical radiation of such a wavelength(s), that is strongly absorbed by blood red cells and/or walls of blood vessels and is reflected and/or scattered from blood vessel surrounding tissues, wherein optical radiation of chosen wavelength can enhance contrast between blood vessels and/or surrounding tissues, thus allowing improved blood vessel visualization for a clinician;
e. puncturing the skin with the blood collection needle **202** of the blood collection assembly **201;**
f. localizing a blood vessel position intracutaneously by monitoring illumination events at the distal end **204** of the blood collection needle **202** and/or by traditional means;
g. performing venipuncture, i.e. penetrating the wall of a blood vessel with a distal end **204** of the blood collection needle **202,** while monitoring illumination events underneath the skin, said events comprise at least one of the following: instant disappearance of illumination from the distal end **204** of the blood collection needle **202,** rapid illumination fading, rapid change in light color or rapid illumination fading along with rapid change in light color, wherein said events take place upon entering the lumen of the blood vessel with the tip of said needle and wherein said events are dependent on the optical radiation wavelength being utilized;
h. observing illumination events in the adapter lock **206;**
i. connecting vacuum blood collection tube to the tube holder **208;**
j. monitoring blood flow into a blood collection tube, while an unobstructed blood flow into the lumen **203** of the blood collection needle **202** causes illumination at the adapter lock **206** element to disappear at once;
k. removing vacuum blood collection tube and tourniquet;
l. removing blood collection assembly **201** from blood vessel;
m. deactivating the optical radiation source **220.**

It is to be understood, that technical solutions disclosed herein in accordance with abovesaid embodiments, while concerning blood collection needle, adapter lock and/or tube holder elements, may be interchangeable and interconnectable in any technically suitable way and are not intended to limit the invention, but to teach those skilled in art to implement the invention within the limits of the protection scope thereof disclosed in independent claims.

## Claims

1. A blood collection needle assembly (2on) comprising:
a. a hollow blood collection needle having a distal end (204), a proximal end (205) and a needle wall that defines a lumen (203) of the needle, wherein a blood entry aperture (204a) is formed at the distal end of the needle and a blood exit aperture is formed at the proximal end of the needle,
b. a tube holder (208) attached to the blood collection needle, and
c. a light source (220);
**characterized in that** the light source (220) is configured to emit light and connected to or integrated in the tube holder (208),
wherein the wall of the blood collection needle (2on) has a light entry aperture (211) which is arranged at a position between the blood entry aperture (204a) and the blood exit aperture, and
wherein the light emitted by the light source (220) is allowed to enter the lumen (203) of the blood collection needle (2on) through the light entry aperture (211).

2. The blood collection needle assembly of claim 1, wherein at least 40% of light contributing to the total light intensity emitted from the distal end of the blood collection needle has a wavelength between 510 nm and 600 nm, preferably at least 30% of light, contributing to the total intensity emitted from the distal end of the blood collection needle has a wavelength selected from one of the following ranges: between 510 nm and 550 nm, or 530 nm and 570 nm, or 560 nm and 600 nm.

3. The blood collection needle assembly of either claim 1 or claim 2, comprising an adapter lock (206) which connects the blood collection needle to the tube holder (208).

4. The blood collection needle assembly of any of the preceding claims, wherein the light entry aperture (211) is closed by a non-diffusing light entry member that is transparent for at least 30% of light emitted by the light source (220).

5. The blood collection needle assembly of claim 4, wherein at least a portion of the light entry member is formed by a light guide (210) extending through the light entry aperture (211), and the light guide (210) preferably further extends along the lumen (203) towards the distal end (204) of the blood collection needle.

6. The blood collection needle assembly of either claim 4 or claim 5, wherein the light guide (210) terminates anywhere in between and/or including the light entry aperture (211) and the blood entry aperture (204a) of the blood collection needle, and/or wherein the light guide (210) extends in between the light entry aperture (211) and the distal end (204) of the blood collection needle and terminates anywhere within the distal end (204).

7. The blood collection needle assembly of any one of claims 4 to 6, wherein the light guide (210) extends in between the light entry aperture (211) and the distal end (204) of the blood collection needle and terminates at a distance of at most 5 mm from the blood entry aperture (204a).

8. The blood collection needle assembly of any one of claims 5 to 7, wherein the light guide (210) has a curved element, wherein the curve angle is between 60° and 175°.

9. A blood collection needle for a blood collection needle assembly (201), wherein the needle comprises
a. a distal end (204),
b. a proximal end (205),
c. a tube wall that defines a lumen (203) of the needle,
d. a blood entry aperture (204a) formed at the distal end (204) of the needle, and
e. a blood exit aperture formed at the proximal end of the needle,
**characterized in that** the blood collection needle further comprises
f. a light entry aperture (211) which is arranged at a position between the blood entry aperture and the blood exit aperture, and
g. a light guide (210) that extends through the light entry aperture and along at least a portion of the lumen towards the blood entry aperture.

10. The blood collection needle of claim 9, wherein the light guide (210) extends until the blood entry aperture (204a) of the blood collection needle.

11. The blood collection needle of claim 9, wherein the light guide (210) terminates anywhere in between and/or including the light entry aperture (211) and the blood entry aperture (204a) of the blood collection needle.

12. The blood collection needle of either claim 9 or claim 11, wherein the light guide (210) extends in between the light entry aperture (211) and the distal end (204) of the blood collection needle and terminates anywhere within the distal end (204).

13. The blood collection needle of any one of claims 9, 11 or 12, wherein the light guide (210) extends in between the light entry aperture (211) and the distal end (204) of the blood collection needle and terminates at a distance of at most 5 mm from the blood entry aperture (204a).

14. The blood collection needle of claim 9, wherein the light guide (210) is an optical fiber, and/or wherein the light guide (210) has a curved element, wherein the curve angle is between 60° and 175°.

15. The blood collection needle of any one of claims 9 to 14, comprising an adapter lock (206) which is configured to connect the blood collection needle to a tube holder (208).

## Patentansprüche

1. Blutentnahmenadelanordnung (201), die Folgendes umfasst:
a. eine hohle Blutentnahmenadel mit einem distalen Ende (204), einem proximalen Ende (205) und einer Nadelwand, die ein Lumen (203) der Nadel definiert, wobei eine Bluteintrittsöffnung (204a) am distalen Ende der Nadel gebildet ist und eine Blutaustrittsöffnung am proximalen Ende der Nadel gebildet ist,
b. einen Röhrenhalter (208), der an der Blutentnahmenadel angebracht ist, und
c. eine Lichtquelle (220);
**dadurch gekennzeichnet, dass** die Lichtquelle (220) dazu konfiguriert ist, Licht auszustrahlen und mit dem Röhrenhalter (208) verbunden oder darin integriert ist,
wobei die Wand der Blutentnahmenadel (201) eine Lichteintrittsöffnung (211) aufweist, die an einer Position zwischen der Bluteintrittsöffnung (204a) und der Blutaustrittsöffnung angeordnet ist, und
wobei dem von der Lichtquelle (220) ausgestrahlten Licht ermöglicht wird, durch die Lichteintrittsöffnung (211) in das Lumen (203) der Blutentnahmenadel (201) einzutreten.

2. Blutentnahmenadelanordnung nach Anspruch 1, wobei mindestens 40 % des zu der vom distalen Ende der Blutentnahmenadel ausgestrahlten Gesamtlichtintensität beitragenden Lichts eine Wellenlänge zwischen 510 nm und 600 nm aufweist und vorzugsweise mindestens 30 % des zu der vom distalen Ende der Blutentnahmenadel ausgestrahlten Gesamtlichtintensität beitragenden Lichts eine aus einem der folgenden Bereiche ausgewählte Wellenlänge aufweist: zwischen 510 nm und 550 nm oder 530 nm und 570 nm oder 560 nm und 600 nm.

3. Blutentnahmenadelanordnung nach Anspruch 1 oder Anspruch 2, umfassend einen Adapterverschluss (206), der die Blutentnahmenadel mit dem Röhrenhalter (208) verbindet.

4. Blutentnahmenadelanordnung nach einem der vorangehenden Ansprüche, wobei die Lichteintrittsöffnung (211) von einem nicht streuenden Lichteintrittselement verschlossen wird, das für mindestens 30 % des von der Lichtquelle (220) ausgestrahltem Lichts transparent ist.

5. Blutentnahmenadelanordnung nach Anspruch 4, wobei mindestens ein Abschnitt des Lichteintrittselements von einem Lichtleiter (210) gebildet wird, der sich durch die Lichteintrittsöffnung (211) erstreckt, und sich der Lichtleiter (210) vorzugsweise weiter entlang dem Lumen (203) auf das distale Ende (204) der Blutentnahmenadel zu erstreckt.

6. Blutentnahmenadelanordnung nach Anspruch 4 oder Anspruch 5, wobei der Lichtleiter (210) an einer beliebigen Stelle zwischen und/oder einschließlich der Lichteintrittsöffnung (211) und der Bluteintrittsöffnung (204a) der Blutentnahmenadel endet, und/oder wobei sich der Lichtleiter (210) zwischen der Lichteintrittsöffnung (211) und dem distalen Ende (204) der Blutentnahmenadel erstreckt und an einer beliebigen Stelle innerhalb des distalen Endes (204) endet.

7. Blutentnahmenadelanordnung nach einem der Ansprüche 4 bis 6, wobei sich der Lichtleiter (210) zwischen der Lichteintrittsöffnung (211) und dem distalen Ende (204) der Blutentnahmenadel erstreckt und in einer Entfernung von höchstens 5 mm von der Bluteintrittsöffnung (204a) endet.

8. Blutentnahmenadelanordnung nach einem der Ansprüche 5 bis 7, wobei der Lichtleiter (210) ein gekrümmtes Element aufweist, wobei der Krümmungswinkel zwischen 60° und 175° beträgt.

9. Blutentnahmenadel für eine Blutentnahmenadelanordnung (201), wobei die Nadel Folgendes umfasst:
a. ein distales Ende (204),
b. ein proximales Ende (205),
c. eine Röhrenwand, die ein Lumen (203) der Nadel definiert,
d. eine Bluteintrittsöffnung (204a), die am distalen Ende (204) der Nadel gebildet ist, und
e. eine Blutaustrittsöffnung, die am proximalen Ende der Nadel gebildet ist, **dadurch gekennzeichnet, dass** die Blutentnahmenadel weiter Folgendes umfasst:
f. eine Lichteintrittsöffnung (211), die an einer Position zwischen der Bluteintrittsöffnung und der Blutaustrittsöffnung angeordnet ist, und
g. einen Lichtleiter (210), der sich durch die Lichteintrittsöffnung und entlang mindestens einem Abschnitt des Lumens auf die Bluteintrittsöffnung zu erstreckt.

10. Blutentnahmenadel nach Anspruch 9, wobei sich der Lichtleiter (210) bis zu der Bluteintrittsöffnung (204a) der Blutentnahmenadel erstreckt.

11. Blutentnahmenadel nach Anspruch 9, wobei der Lichtleiter (210) an einer beliebigen Stelle zwischen und/oder einschließlich der Lichteintrittsöffnung (211) und der Bluteintrittsöffnung (204a) der Blutentnahmenadel endet.

12. Blutentnahmenadel nach Anspruch 9 oder Anspruch 11, wobei sich der Lichtleiter (210) zwischen der Lichteintrittsöffnung (211) und dem distalen Ende (204) der Blutentnahmenadel erstreckt und an einer beliebigen Stelle innerhalb des distalen Endes (204) endet.

13. Blutentnahmenadel nach einem der Ansprüche 9, 11 oder 12, wobei sich der Lichtleiter (210) zwischen der Lichteintrittsöffnung (211) und dem distalen Ende (204) der Blutentnahmenadel erstreckt und in einer Entfernung von höchstens 5 mm von der Bluteintrittsöffnung (204a) endet.

14. Blutentnahmenadel nach Anspruch 9, wobei es sich bei dem Lichtleiter (210) um eine optische Faser handelt, und/oder wobei der Lichtleiter (210) ein gekrümmtes Element aufweist, wobei der Krümmungswinkel zwischen 60° und 175° beträgt.

15. Blutentnahmenadel nach einem der Ansprüche 9 bis 14, umfassend einen Adapterverschluss (206), der dazu konfiguriert ist, die Blutentnahmenadel mit einem Röhrenhalter (208) zu verbinden.

## Revendications

1. Ensemble aiguille de prélèvement sanguin (201), comprenant :
a) une aiguille de prélèvement sanguin creuse qui possède une extrémité distale (204), une extrémité proximale (205) et une paroi d'aiguille qui définit un conduit (203) de l'aiguille, un orifice d'entrée de sang (204a) étant formé à l'extrémité distale de l'aiguille et un orifice de sortie de sang étant formé à l'extrémité proximale de l'aiguille,
b) un support de tube (208) fixé à l'aiguille de prélèvement sanguin, et
c) une source de lumière (220) ;
**caractérisé en ce que** la source de lumière (220) est configurée pour émettre de la lumière et est raccordée à ou intégrée dans le support de tube (208),
dans lequel la paroi de l'aiguille de prélèvement sanguin (201) possède un orifice d'entrée de lumière (211) qui est disposé à une position entre l'orifice d'entrée de sang (204a) et l'orifice de sortie de sang, et
dans lequel la lumière émise par la source de lumière (220) peut pénétrer dans le conduit (203) de l'aiguille de prélèvement sanguin (201) par l'orifice d'entrée de lumière (211).

2. Ensemble aiguille de prélèvement sanguin selon la revendication 1, dans lequel au moins 40 % de la lumière qui contribue à l'intensité totale de la lumière émise depuis l'extrémité distale de l'aiguille de prélèvement sanguin a une longueur d'onde entre 510 nm et 600 nm, de préférence au moins 30 % de la lumière qui contribue à l'intensité totale émise depuis l'extrémité distale de l'aiguille de prélèvement sanguin a une longueur d'onde choisie dans une des gammes suivantes : entre 510 nm et 550 nm, ou entre 530 nm et 570 nm, ou entre 560 nm et 600 nm.

3. Ensemble aiguille de prélèvement sanguin selon la revendication 1 ou la revendication 2, comprenant un verrouillage adaptateur (206) qui raccorde l'aiguille de prélèvement sanguin au support de tube (208).

4. Ensemble aiguille de prélèvement sanguin selon l'une quelconque des revendications précédentes, dans lequel l'orifice d'entrée de lumière (211) est fermé par un élément d'entrée de lumière non diffusant qui est transparent pour au moins 30 % de la lumière émise par la source de lumière (220).

5. Ensemble aiguille de prélèvement sanguin selon la revendication 4, dans lequel une partie au moins de l'élément d'entrée de lumière est formé par un guide de lumière (210) qui s'étend à travers l'orifice d'entrée de lumière (211) et, de préférence, le guide de lumière (210) s'étend en outre le long du conduit (203) en direction de l'extrémité distale (204) de l'aiguille de prélèvement sanguin.

6. Ensemble aiguille de prélèvement sanguin selon la revendication 4 ou la revendication 5, dans lequel le guide de lumière (210) se termine à un endroit quelconque entre et/ou incluant l'orifice d'entrée de lumière (211) et l'orifice d'entrée de sang (204a) de l'aiguille de prélèvement sanguin, et/ou dans lequel le guide de lumière (210) s'étend entre l'orifice d'entrée de lumière (211) et l'extrémité distale (204) de l'aiguille de prélèvement sanguin et se termine à un endroit quelconque à l'intérieur de l'extrémité distale (204).

7. Ensemble aiguille de prélèvement sanguin selon l'une quelconque des revendications 4 à 6, dans lequel le guide de lumière (210) s'étend entre l'orifice d'entrée de lumière (211) et l'extrémité distale (204) de l'aiguille de prélèvement sanguin et se termine à une distance au maximum égale à 5 mm de l'orifice d'entrée de sang (204a).

8. Ensemble aiguille de prélèvement sanguin selon l'une quelconque des revendications 5 à 7, dans lequel le guide de lumière (210) possède un élément incurvé, l'angle de courbure allant de 60° à 175°.

9. Aiguille de prélèvement sanguin destinée à un ensemble aiguille de prélèvement sanguin (201), ladite aiguille comprenant :
a) une extrémité distale (204),
b) une extrémité proximale (205),
c) une paroi de tube qui définit un conduit (203) de l'aiguille,
d) un orifice d'entrée de sang (204a) formé à l'extrémité distale (204) de l'aiguille, et
e) un orifice de sortie de sang formé à l'extrémité proximale de l'aiguille,
**caractérisée en ce que** l'aiguille de prélèvement sanguin comprend en outre
f) un orifice d'entrée de lumière (211) qui est disposé à une position entre l'orifice d'entrée de sang et l'orifice de sortie de sang, et
g) un guide de lumière (210) qui s'étend à travers l'orifice d'entrée de lumière et le long d'une partie au moins du conduit en direction de l'orifice d'entrée de sang.

10. Aiguille de prélèvement sanguin selon la revendication 9, dans laquelle le guide de lumière (210) s'étend jusqu'à l'orifice d'entrée de sang (204a) de l'aiguille de prélèvement sanguin.

11. Aiguille de prélèvement sanguin selon la revendication 9, dans laquelle le guide de lumière (210) se termine à un endroit quelconque entre et/ou incluant l'orifice d'entrée de lumière (211) et l'orifice d'entrée de sang (204a) de l'aiguille de prélèvement sanguin.

12. Aiguille de prélèvement sanguin selon la revendication 9 ou la revendication 11, dans laquelle le guide de lumière (210) s'étend entre l'orifice d'entrée de lumière (211) et l'extrémité distale (204) de l'aiguille de prélèvement sanguin et se termine à un endroit quelconque à l'intérieur de l'extrémité distale (204).

13. Aiguille de prélèvement sanguin selon l'une quelconque des revendications 9, 11 ou 12, dans laquelle le guide de lumière (210) s'étend entre l'orifice d'entrée de lumière (211) et l'extrémité distale (204) de l'aiguille de prélèvement sanguin et se termine à une distance au maximum égale à 5 mm de l'orifice d'entrée de sang (204a).

14. Aiguille de prélèvement sanguin selon la revendication 9, dans laquelle le guide de lumière (210) est une fibre optique, et/ou dans laquelle le guide de lumière (210) possède un élément incurvé, l'angle de courbure allant de 60° à 175°.

15. Aiguille de prélèvement sanguin selon l'une quelconque des revendications 9 à 14, comprenant un verrouillage adaptateur (206) qui est configuré pour raccorder l'aiguille de prélèvement sanguin à un support de tube (208) .
